# EUROPEAN PATENT SPECIFICATION

(11) **EP 1 351 911 B1**
(45) Date of publication and mention of the grant of the patent: **26.07.2006**
(21) Application number: 01271340.0
(22) Date of filing: 14.12.2001
(51) Int. Cl.: C07C 41/30, C07C 43/23

(54) **MATERIALS AND METHODS FOR SYNTHESIZING STILBENES**
MATERIALIEN UND VERFAHREN ZUR HERSTELLUNG VON STILBENEN
SUBSTANCES ET METHODES DE SYNTHESE DE STILBENES

(30) Priority: 21.12.2000 GB 0031263
(43) Date of publication of application: 15.10.2003
(73) Proprietor: Cancer Research Ventures Limited, London WC2A 3NL (GB)
(72) Inventor: HADFIELD, John A., Paterson Inst. for Cancer Res., Manchester, Greater Manchester M20 4BX (GB); MCGOWN, Alan T., Paterson Inst. for Cancer Res., Manchester, Greater Manchester M20 4BX (GB); GAUKROGER, Keira, Paterson Inst. for Cancer Res., Manchester, Greater Manchester M20 4BX (GB); HEPWORTH, Lucy, Annette,, Lymm, Cheshire, WA 13 9QS (GB); LAWRENCE, Nicholas James, c/o Dept. of Chemistry, Cardiff CF10 3TB (GB)
(74) Representative: Kiddle, Simon John
(86) International application number: PCT/GB2001/005534
(87) International publication number: WO 2002/049994

(56) References cited:
- W. J. WARD, JR.: "Metal ion effects in Wittig reactions: a general hypothesis for the mechanism of the Wittig reaction" JOURNAL OF ORGANIC CHEMISTRY, vol. 55, no. 2, 19 January 1990 (1990-01-19), pages 493-500, XP002196096 EASTON US
- R. M. LETCHER: "Chemical constituents of the Combretaceae. Part. II. Substituted phenanthrenes and 9,10-dihydrophenanthrenes and a substituted bibenzyl from the heartwood of Combretum molle" JOURNAL OF THE CHEMICAL SOCIETY, PERKIN TRANSACTIONS 1, 1972, pages 206-210, XP002196097 LETCHWORTH GB
- R. M. LETCHER: "Chemical constituents of the Combretaceae. Part III. Substituted phenanthrenes, 9,10-dihydrophenanthrenes, and bibenzyls from the heartwood of Combretum psidioides" JOURNAL OF THE CHEMICAL SOCIETY, PERKIN TRANSACTIONS 1, 1972, pages 2941-2946, XP002196098 LETCHWORTH GB
- DATABASE CROSSFIRE BEILSTEIN [Online] Beilstein Institut zur Förderung der chemischen Wissenschaft, Frankfurt am Main, DE; XP002196099 & YAKUGAKU ZASSHI, vol. 64, 1944, pages 164-167,

## Description

### Field of the Invention

The present invention relates materials and methods for synthesizing stilbenes, and in particular to processes for the synthesis of substituted stilbenes such as combretastatin.

### Background of the Invention

The stilbene *cis*-combretastatin A-4, isolated from the African bush willow, Combretum caffrum shows exciting potential as an anticancer agent, binding strongly to tubulin and displaying potent and selective toxicity toward tumour vasculature. Cis-combretastatin A-4 is able to inhibit cell growth at low concentrations (IC₅₀, P388 murine leukaemia cell line 2.6 nM). The potency of trans-combretastatin A-4 is much lower and inhibits cell growth in the millimolar range. The isolation of cis-combretastatin A-4 is reported in US Patent No:4,996,237 (Arizona Board of Regents).

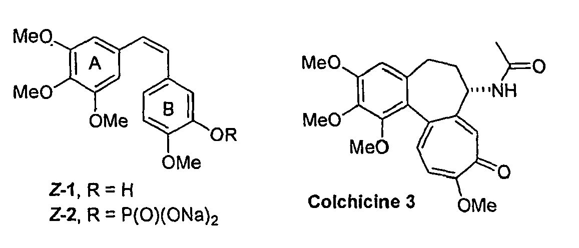

However, the low solubility of cis-combretastatin A-4 in water and saline has led to attempts in the art to make related compounds or prodrugs which retain the activity of cis-combretastatin A-4 as an anticancer agent and which have enhanced solubility. These attempts focus on forming salts or derivatives at the phenolic hydroxyl group of combretastatin. By way of example:

US Patent No: 5, 561, 122 (Arizona Board of Regents) which discloses the sodium and potassium salts of cis-combretastatin A-4 and a hemisuccinic acid ester derivative.

WO 99/35150 (Arizona Board of Regents) which discloses the lithium, caesium, magnesium, calcium, manganese and zinc salts of *cis*-combretastatin A-4, and ammonium cation salts with imidazole, morpholine, piperazine, piperidine, pyrazole, pyridine, adenosine, cinchonine, glucosamine, quinine, quinidine, tetracycline and verapamil.

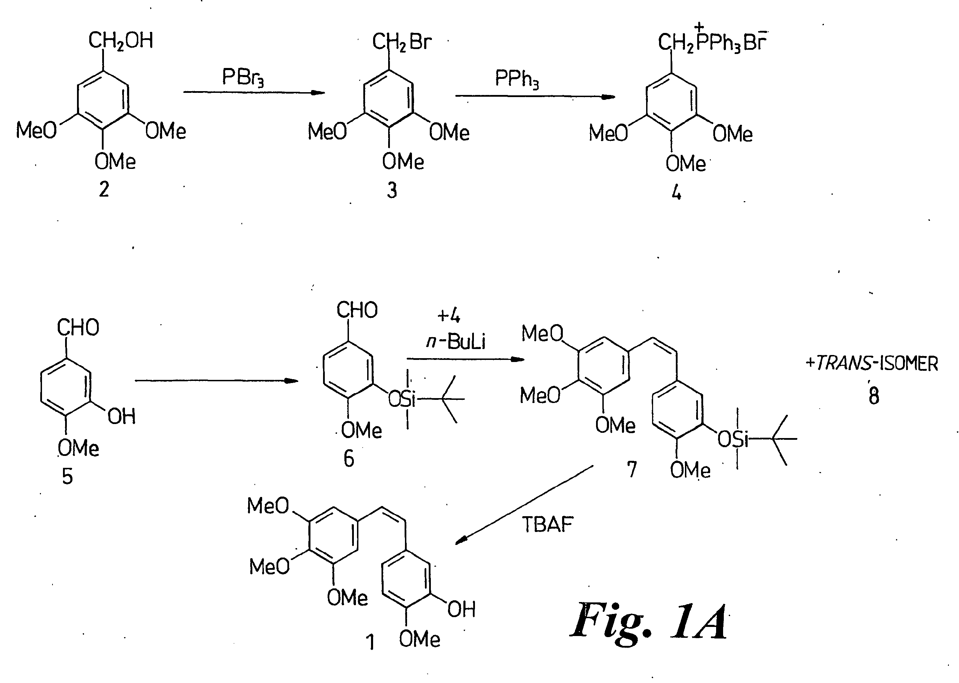

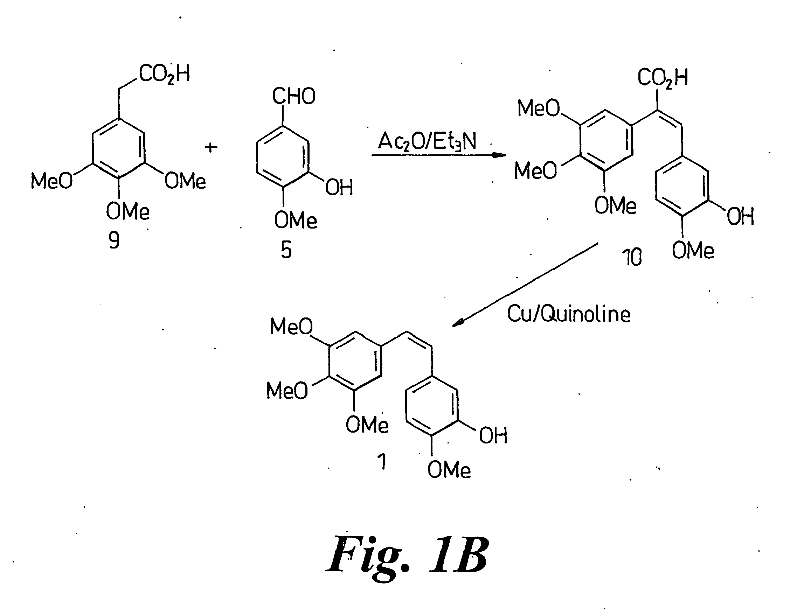

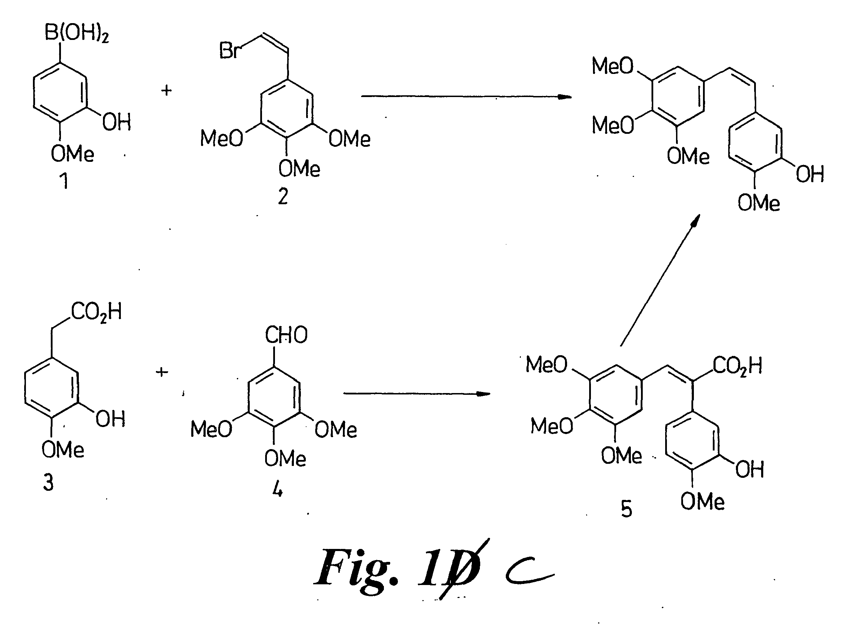

The Wittig reaction is a useful method for the synthesis of combretastatins that has been employed in the past, see for example WO92/16486 (Aston Molecules Limited). However, one significant disadvantage is that a mixture of Z- and E-isomers are produced, thus reducing the yield of the desired Z-isomer. Also, the synthesis of combretastatin A-4 (1) requires five synthetic steps (see Figure 1) and a difficult chromatographic separation of the two silyl protected stilbene isomers (7,8).

*J. Chem. Soc., Perkin Transactions I,* (1972) 206-210 describes the formation of acetoxy- and methoxy substituted stilbenes by a Perkin condensation.

*J*. *Chem. Soc., Perkin Transactions I,* (1972) 2941-2946 describe the formation of acetoxy-methoxy-substituted stilbenes by a Perkin condensation.

Accordingly, despite the efforts in the art, it remains a significant problem in synthesizing substituted stilbenes such as combretastatin.

### Summary of the Invention

Broadly, the present invention relates to new methods for producing substituted stilbenes such as combretastatin A4. The present invention relates in particular to methods which are stereoselective for the cis isomer of the substituted stilbene.

The present invention therefore provides a method of synthesis which is stereospecific for cis-stilbenes. In one aspect, the present invention employs a Perkin-type condensation of an arylacetic acid and a substituted benzaldehyde, followed by a decarboxylation reaction to produce the substituted *cis*-stilbenes.

In preferred embodiments of the invention, in addition to the selectivity described above the methods may result in syntheses with reduced numbers of steps as compared to the prior art methods and/or syntheses which employ inexpensive reagents.

Accordingly, the present invention provides a method for synthesizing a cis-stilbene represented by the general formula: 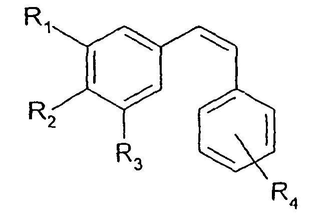 the method comprising:
reacting an arylacetic acid represented by general formula: 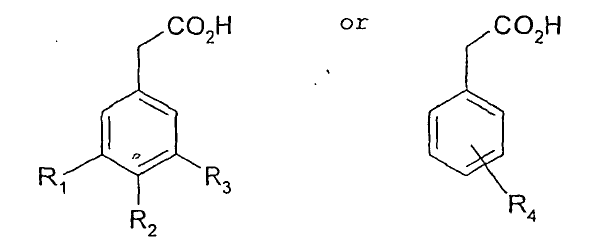
with a substituted benzaldehyde represented by general formula: 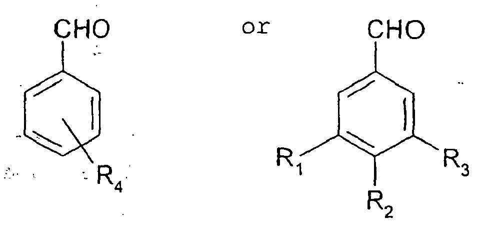
to form a condensation product and decarboxylating the condensation product in the presence of a copper catalyst to produce the cis-stilbene.

R₁, R₂ and R₃ are independently selected from alkyl, alkoxy, halogen or SR groups, and more preferably, methyl, ethyl, methoxy, ethoxy or fluoro groups.

In the above formula, preferably R₄ is one, two or three substituents at the or 4-position, or at the 3-position and the 4-position, or at the 3-position, the 4-position and the 5-position of the benzaldehyde. Where multiple substituents are present they may be the same or different.

The R₄ substituent or substituents are selected from hydrogen, hydroxyl, halogen or alkoxy groups.

For the synthesis of *cis*-combretastatin-A4, R₁, R₂, and R₃ are all methoxy, and R₄ is a hydroxyl group at the 3-position and a methoxy group at the 4-position.

In the present invention, where alkyl or other alkyl containing substituents such as alkoxy are employed, the alkyl groups can be straight chain or branched and are preferably C₁₋₁₀.

This Perkin-type reaction described herein has the advantage that it can be used for the synthesis of cis-combretastatin A-4 in fewer steps that the prior art Wittig reaction and from readily available starting materials. The reaction is also stereoselective and is tolerant of the identity of the R groups present on the arylacetic acid and the substituted benzaldehyde.

Preferably, the initial condensation reaction is carried out in the presence of a carboxylic acid anhydride (either a mixed or symmetrical anhydride) and a tertiary amine. Preferred examples of these reagents are acetic anhydride and triethylamine, added simultaneously or sequentially to the reaction mixture. Typically, the reaction is conducted by heating the reagents under reflux in a solvent, the identity of which can be readily determined by the skilled person. Preferred reaction times are between 1 and 6 hours, preferably about 3 hours. Optionally, the substituted prop-2-enoic acid product is recrystallised prior to continuing to the decarboxylation reaction.

Conveniently, the decarboxylation reaction can be carried out by heating, optionally in the presence of a copper catalyst, typically to a temperature between about 200 and 250°C. Examples of copper catalyst include powdered copper or copper compounds such as copper triflate or copper chromite. The reaction is conveniently.carried out in a solvent with a high boiling point such as aromatic pyridine-like bases, e.g. quinoline, substituted quinolines or isoquinolines or similar bases. Preferred reaction conditions for this step involve employing a powdered copper catalyst in a solvent such as quinoline at a temperature of about 230°C.

In some embodiments, the method may comprise the initial steps of synthesizing the arylacetic acid or the substituted benzaldehyde, e.g. using methods available in the literature.

The method of the invention may comprise the further step of reacting the stilbene (either a Z-stilbene or an E-stilbene) to form a derivative, salt or prodrug. This might be done to improve the properties of compounds used as pharmaceuticals, e.g. to modify their solubility or other pharmacological properties. Preferred salts and derivatives are produced by reaction of the hydroxyl group on the second ring of the stilbene and are discussed in more detail below.

The method of the invention may further comprise purifying the stilbene product, or a derivative or salt thereof, and optionally formulating it as a composition, e.g. for pharmaceutical use.

Embodiments of the present invention will now be described in more detail by way of example and not limitation with reference to the accompanying drawings.

### Brief Description of the Figures

Figure 1A shows a prior art synthesis of *cis-*combretastatin A4 using the Wittig reaction.
Figure 1B shows the synthesis of Z-stilbenes using a Perkin condensation and decarboxylation reaction.
Figure 1C shows the synthesis of stilbenes using an alternative Perkin/decarboxylation method.

### Detailed Description

### Pharmaceutical Compositions

The compounds of the invention may be derivatised in various ways. As used herein "derivatives" of the compounds includes salts, esters such as in vivo hydrolysable esters, free acids or bases, hydrates, prodrugs or coupling partners. In the case of compounds which are combretastatin or analogues thereof, preferably the derivatives are soluble in water and/or saline or can be hydrolysed to provide soluble, and therefore physiologically accessible active agent.

Examples in the prior art of salts or prodrugs of cis-combretastatin A-4 focus on forming salts or derivatives at the phenolic hydroxyl group of combretastatin. These include sodium phosphate salts, sodium and potassium salts (US Patent No: 5,561,122), lithium, caesium, magnesium, calcium, manganese and zinc salts of cis-combretastatin A-4, and ammonium cation salts with imidazole, morpholine, piperazine, piperidine, pyrazole, pyridine, adenosine, cinchonine, glucosamine, quinine, quinidine, tetracycline and verapamil (WO99/35150).

Salts of the compounds of the invention are preferably physiologically well tolerated and non toxic. Many examples of salts are known to those skilled in the art. Compounds having acidic groups, can form salts with alkaline or alkaline earth metals such as Na, K, Mg and Ca, and with organic amines such as triethylamine and Tris (2-hydroxyethyl)amine. Salts can be formed between compounds with basic groups, e.g. amines, with inorganic acids such as hydrochloric acid, phosphoric acid or sulfuric acid, or organic acids such as acetic acid, citric acid, benzoic acid, fumaric acid, or tartaric acid. Compounds having both acidic and basic groups can form internal salts.

Esters can be formed between hydroxyl or carboxylic acid groups present in the compound and an appropriate carboxylic acid or alcohol reaction partner, using techniques well known in the art. Examples of esters include those formed between the phenolic hydroxyl of the substituted stilbenes and carboxylic acids, hemisuccinic acid esters, phosphate esters, sulphate esters and selenate esters.

Derivatives which as prodrugs of the compounds are convertible *in vivo* or in vitro into one of the parent compounds. Typically, at least one of the biological activities of compound will be reduced in the prodrug form of the compound, and can be activated by conversion of the prodrug to release the compound or a metabolite of it. Examples of prodrugs include combretastatin A1 phosphate, combretastatin A4 phosphate and RH1 (2,5,-diaziridinyl-3-(hydroxymethyl)-6-methyl-1,4-benzoquinone.

Other derivatives include coupling partners of the compounds in which the compounds is linked to a coupling partner, e.g. by being chemically coupled to the compound or physically associated with it. Examples of coupling partners include a label or reporter molecule, a supporting substrate, a carrier or transport molecule, an effector, a drug, an antibody or an inhibitor. Coupling partners can be covalently linked to compounds of the invention via an appropriate functional group on the compound such as a hydroxyl group, a carboxyl group or an amino group.

The compounds described herein or their derivatives can be formulated in pharmaceutical compositions, and administered to patients in a variety of forms, in particular to treat conditions which are ameliorated by the activation of the compound.

Pharmaceutical compositions for oral administration may be in tablet, capsule, powder, cream, liquid form or encapsulated by liposomes. A tablet may include a solid carrier such as gelatin or an adjuvant or an inert diluent. Liquid pharmaceutical compositions generally include a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1wt% of the compound.

Parental administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes. For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicizing agent, preservative or antioxidant or other materials well known to those skilled in the art. Such materials should be non-toxic and should not interfere with the efficacy of the active ingredient. The precise nature of the carrier or other material may depend on the route of administration, e.g. orally or parentally.

Liquid pharmaceutical compositions are typically formulated to have a pH between about 3.0 and 9.0, more preferably between about 4.5 and 8.5 and still more preferably between about 5.0 and 8.0. The pH of a composition can be maintained by the use of a buffer such as acetate, citrate, phosphate, succinate, Tris or histidine, typically employed in the range from about 1 mM to 50 mM. The pH of compositions can otherwise be adjusted by using physiologically acceptable acids or bases.

Preservatives are generally included in pharmaceutical compositions to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalconium chloride and benzethonium chloride. Preservatives are typically employed in the range of about 0.1 to 1.0 % (w/v).

Preferably, the pharmaceutically compositions are given to an individual in a "prophylactically effective amount" or a "therapeutically effective amount" (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980. By way of example, and the compositions are preferably administered to patients in dosages of between about 0.01 and 100mg of active compound per kg of body weight, and more preferably between about 0.5 and 10mg/kg of body weight.

The compounds may be used in the treatment of cancer and other conditions involving abnormal proliferation of vasculature including diabetic retinopathy, psoriasis and endometriosis.

### General

Proton nuclear magnetic resonance (¹H NMR) spectra were recorded on a Brüker AC 300 (300 MHz) or AC 400 (400 MHz) NMR spectrometer. Chemical shifts, δ, for all NMR spectra are given in ppm, relative to tetramethylsilane, and, unless otherwise stated, using CDCl₃ as both solvent and internal standard. Coupling constants (J) were measured in Hz. Melting points were determined on a Gallenkamp melting point apparatus and are uncorrected. The UV/VIS spectra were determined using a Hewlett-Packard HP8452 diode-array spectrophotometer. Extinction coefficients (ε) are presented as their natural logarithms. Microanalyses were carried out by the microanalytical laboratory, Department of Chemistry, University of Manchester. High resolution mass spectroscopy was determined using a Kratos Concept 15 mass spectrometer. Thin layer chromatography (tlc) was performed using precoated aluminium-backed silica gel plates (60 F₂₅₄) with 0.2 mm thickness (Merck), with observation under UV when necessary. Gas chromatography was carried out using an SE 54 column at 195-225 kPa at 1.5kPa/min. The oven temperature was 180-280°C at 5°C/min.

### 1. Stereoselective Synthesis of Z-stilbenes

A general method for the synthesis of Z-stilbenes involves the copper-catalysed decarboxylation of an E-2,3-diarylacrylic acid. These acids are prepared by the Perkin-type condensation of an arylacetic acid with a benzaldehyde. However, this two-step methodology of preparing stilbenes has not been applied to the synthesis of combretastatin A-4 (1). In our laboratory the reaction of 3,4,5-trimethoxyphenylacetic acid (9) with 3-hydroxy-4-methoxybenzaldehyde (5) afforded *E*-3-(3'-hydroxy-4'-methoxyphenyl)-2-(3'',4'',5''-trimethoxyphenyl)prop-2-enoic acid (10) in 60% yield. Decarboxylation of this acid (10) was achieved by heating with copper powder in quinoline at 230°C. The desired combretastatin A-4 (1) was isolated in ca 70% yield (Figure 1B) after purification by chromatography or by recrystallisation. The overall yield for this two-step synthesis is 41% using inexpensive reagents. The previously published non-stereoselective five-step procedure for the synthesis of combretastatin A-4 (1) yields 31-45% depending on the scale of the reactions.

### E)-3-(3'-Hydroxy-4'-methoxyphenyl)-2-(3'',4'',5''-trimethoxyphenyl)-prop-2-enoic acid (10)

A solution of 3-hydroxy-4-methoxy-benzaldehyde (5) (0.67 g, 4.4 mmol), 3,4,5-trimethoxyphenylacetic acid (9) (2 g, 8.84 mmol) in acetic anhydride (4 ml) and triethylamine (2 ml) were heated under reflux for 3 h. After careful addition of concentrated hydrochloric acid (6 ml), the resulting solid was filtered off and recrystallised from ethanol to give the title acid (10) (950 mg, 2.63 mmol, 60%) as fine yellow needles. m.p. 237-9°C. δ_{H} (300 MHz d₆-DMSO) : 3.68 (6 H, s, 2 x OCH₃) ; 3.72 (3 H, s, OCH₃); 3.74 (3 H, s, OCH₃) ; 6.44 (2 H, s, H-2", 6"); 6.54 (1 H, d, *J =* 1. 9, H-2') ; 6.61 (1 H, dd, *J =* 8.3, 1. 9, H-6'); 6.80 (1 H, d, *J* = 8.3, H-5') ; 7.58 (1 H, s, olefinic H).

### Z-1-(3'-Hydroxy-4'-methoxyphenyl)-2-(3'',4'',5''-trimethoxyphenyl)ethene - Combretastatin A-4 (1)

(*E*)-3- (3' -hydroxy-4' -methoxyphenyl)-2- (3'', 4'', 5' '-trimethoxyphenyl)-prop-2-enoic acid (10) (2 g, 5.56 mmol) was added to powdered copper (1.84 g, 28.8 mmol) in quinoline (20 ml, 21.9 g, 0.17 mmol) and the resulting mixture was heated at 200°C for 2 h. Upon cooling, ether was added and the copper filtered off through celite.

The filtrate was washed with concentrated hydrochloric acid (20 ml) and the aqueous layer was separated and extracted with ether (3 x 50 ml). The combined organic layers were washed with saturated aqueous sodium carbonate (50 ml), water (2 x 50 ml), brine (50 ml), dried (MgSO₄) and concentrated *in vacuo.* Flash column chromatography (petrol/EtOAc 7:3) afforded combretastatin A-4 (1) as a pale yellow crystalline solid (1.19 g, 3.77 mmol, 68%). m.p. 117-8°C (lit. 116°C). R_{f} = 0.46 (petrol/EtOAc 1:1) ; δ_{H} (300 MHz): 3.72 (6 H, s, 2 x OCH₃) ; 3.68 (3 H, s, OCH₃) ; 3.89 (3 H, s, OCH₃) ; 5.53 (1 H, s, OH); 6.42 (1 H, d, *J =* 12.4, olefinic H); 6.49 (1 H, d, *J* = 12.4, olefinic H); 6.55 (2 H, s, H-2" , 6''); 6.75 (1 H, d, *J =* 8.3, H-5') ; 6.82 (1 H, dd, *J =* 8.3, 1.9 , H-6'); 6.94 (1 H, d, *J =* 1.9, H-2') .

GC analysis of the crude reaction mixture (without chromatography) showed a ratio of 88:12, cis to *trans,* but following recrystallisation this changed to 98:2. Analysis following flash column chromatography, without recrystallisation, showed the ratio of cis to trans was 99.4:0.6.

## Claims

1. A method for synthesizing a cis-stilbene represented by the general formula: 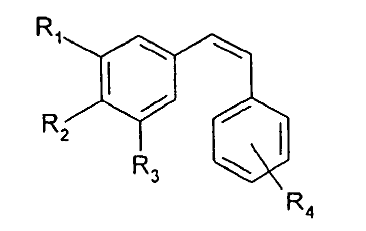 the method comprising:
reacting an arylacetic acid represented by general formula: 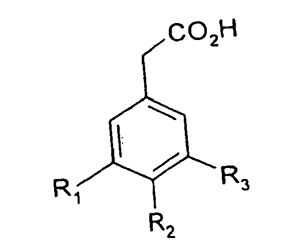 or 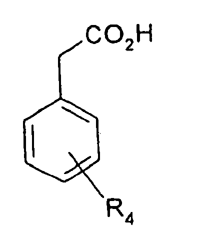
with a substituted benzaldehyde represented by the general formula: 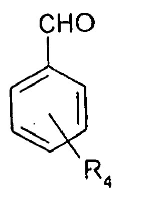 or 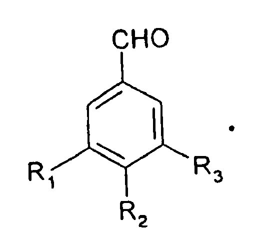
to form a condensation product and decarboxylating the condensation product in the presence of a copper catalyst to produce the cis-stilbene;
wherein the R₁, R₂ and R₃ groups are independently selected from alkyl, alkoxy, halogen or SR, where R is a substituted or unsubstituted alkyl group; and
R₄ is a substituent or substituents at the 4-position, or at the 3-position and the 4-position, or at the 3-position, the 4-position and the 5-position; and
the R₄ substituent or substituents are selected from hydrogen, hydroxyl, halogen or alkoxy groups.

2. The method of claim 1, wherein R₁, R₂ and R₃ are independently selected from methyl, ethyl, methoxy, ethoxy or fluoro groups.

3. The method of claim 1 or claim 2, wherein for the synthesis of *cis*-combretastatin-A4, R₁, R₂, and R₃ are all methoxy, and R₄ is a hydroxyl group at the 3-position and a methoxy group at the 4-position.

4. The method of any preceding claims, wherein the reaction to produce the condensation product is carried out in the presence of a carboxylic acid anhydride and a tertiary amine.

5. The method of claim 4, wherein the carboxylic acid anhydride and the tertiary amine are acetic anhydride and triethylamine, added simultaneously or sequentially to the reaction mixture.

6. The method of any preceding claims, wherein the reaction to produce the condensation product is conducted by heating the reagents under reflux in a solvent.

7. The method of claim 6, wherein the reaction time is between 1 and 6 hours.

8. The method of any one of the preceding claims, wherein the condensation product is is recrystallised prior to carrying out the decarboxylation reaction.

9. The method of any one of the preceding claims, wherein the decarboxylation reaction is carried out by heating to a temperature between about 200 and 250°C.

10. The method of any one of the preceding claims, wherein in the decarboxylation reaction, the condensation product is heated in the presence of the copper catalyst.

11. The method of claim 10, wherein the copper catalyst is powdered copper or a copper compound such as copper triflate or copper chromite.

12. The method of any one of claims 9 to 11, wherein the decarboxylation reaction is carried out in a high boiling point solvent.

13. The method of claim 12, wherein the high boiling point solvent is a quinoline, a substituted quinoline or an isoquinoline.

14. The method of any one of the preceding claims, wherein the decarboxylation reaction uses a powdered copper catalyst in a solvent such as quinoline at a temperature of about 230°C.

15. The method of any one of the preceding claims, which comprises the initial step of synthesizing the arylacetic acid or the substituted benzaldehyde.

16. The method of any one of the preceding claims, further comprising the step of reacting the stilbene to form a derivative, salt or prodrug.

17. The method of any one of the preceding claims, further comprising purifying the stilbene or a derivative or salt thereof.

18. The method of claim 17, further comprising formulating the stilbene in a pharmaceutical composition.

## Patentansprüche

1. Verfahren zum Synthetisieren eines cis-Stilbens der allgemeinen Formel: 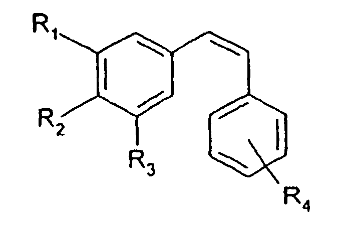 umfassend:
das Umsetzen einer Arylessigsäure der allgemeinen Formel: 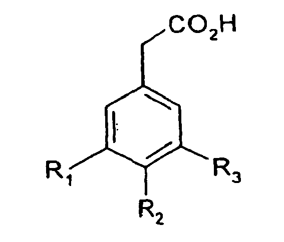 oder 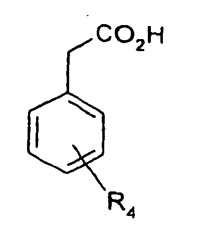
mit einem substituierten Benzaldehyd mit der allgemeinen Formel: 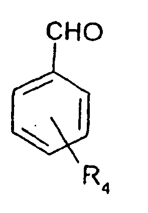 oder 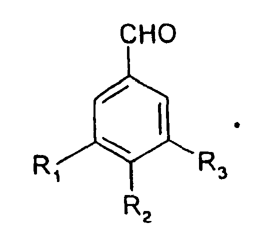 um ein Kondensationsprodukt zu bilden, und Decarboxylierung des Kondensationsprodukt in Gegenwart eines Kupferkatalysators, um das cis-Stilben herzustellen;
worin die Gruppen R₁, R₂ und R₃ unabhängig voneinander aus Alkyl, Alkoxy, Halogen oder SR ausgewählt sind, worin R eine substituierte oder unsubstituierte Alkylgruppe ist; und
R₄ ein Substituent oder Substituenten an der 4-Position oder an der 3-Position und der 4-Position ist bzw. sind; und
der oder die Substituent(en) R₄ aus Wasserstoff-, Hydroxyl-, Halogen- oder Alkoxygruppen ausgewählt ist bzw. sind.

2. Verfahren nach Anspruch 1, worin R₁, R₂ und R₃ unabhängig voneinander aus Methyl-, Ethyl-, Methoxy-, Ethoxy- und Fluorgruppen ausgewählt sind.

3. Verfahren nach Anspruch 1 oder Anspruch 2, worin zur Synthese von cis-Combretastatin-A4 R₁, R₂ und R₃ allesamt Methoxy sind und R₄ eine Hydroxylgruppe an der 3-Position und eine Methoxygruppe an der 4-Position ist.

4. Verfahren nach einem der vorangehenden Ansprüche, worin die Reaktion zur Herstellung des Kondensationsprodukts in Gegenwart eines Carbonsäureanhydrids und eines tertiären Amins durchgeführt wird.

5. Verfahren nach Anspruch 4, worin das Carbonsäureanhydrid und das tertiäre Amin Essigsäureanhydrid und Triethylamin sind, die gleichzeitig oder nacheinander zum Reaktionsgemisch zugesetzt werden.

6. Verfahren nach einem der vorangehenden Ansprüche, worin die Reaktion zur Herstellung des Kondensationsprodukts durch Rückflusserhitzen der Reagenzien in einem Lösungsmittel durchgeführt wird.

7. Verfahren nach Anspruch 6, worin die Reaktionsdauer zwischen 1 und 6 Stunden beträgt.

8. Verfahren nach einem der vorangehenden Ansprüche, worin das Kondensationsprodukt vor Durchführung der Decarboxylierungsreaktion umkristallisiert wird.

9. Verfahren nach einem der vorangehenden Ansprüche, worin die Decarboxylierungsreaktion durch Erhitzen auf eine Temperatur zwischen etwa 200 und 250 °C durchgeführt wird.

10. Verfahren nach einem der vorangehenden Ansprüche, worin das Kondensationsprodukt bei der Decarboxylierungsreaktion in Gegenwart des Kupferkatalysators erhitzt wird.

11. Verfahren nach Anspruch 10, worin der Kupferkatalysator Kupfer in Pulverform oder eine Kupferverbindung, wie z.B. Kupfertriflat oder Kupferchromat(III), ist.

12. Verfahren nach einem der Ansprüche 9 bis 11, worin die Decarboxylierungsreaktion in einem hochsiedenden Lösungsmittel durchgeführt wird.

13. Verfahren nach Anspruch 12, worin das hochsiedende Lösungsmittel ein Chinolin, ein substituiertes Chinolin oder ein Isochinolin ist.

14. Verfahren nach einem der vorangehenden Ansprüche, worin bei der Decarboxylierungsreaktion ein Kupferpulverkatalysator in einem Lösungsmittel, wie z.B. Chinolin, bei einer Temperatur von etwa 230 °C eingesetzt wird.

15. Verfahren nach einem der vorangehenden Ansprüche, das den anfänglichen Schritt der Synthese der Arylessigsäure oder des substituierten Benzaldehyds umfasst.

16. Verfahren nach einem der vorangehenden Ansprüche, weiters umfassend den Schritt des Umsetzens des Stilbens, um ein Derivat, Salz oder Prodrug zu bilden.

17. Verfahren nach einem der vorangehenden Ansprüche, weiters umfassend das Reinigen des Stilbens oder eines Derivats oder Salzes davon.

18. Verfahren nach Anspruch 17, weiters umfassend das Formulieren des Stilbens zu einer pharmazeutischen Zusammensetzung.

## Revendications

1. Méthode pour la synthèse d'un cis-stilbène représenté par la formule générale: 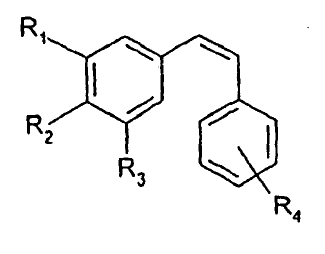 la méthode comprenant:
la réaction d'un acide arylacétique représenté par la formule générale: 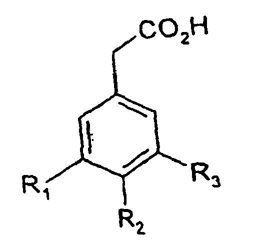 ou 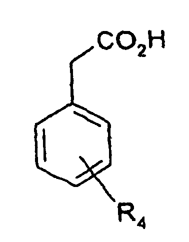
avec un benzaldéhyde substitué représenté par la formule générale: 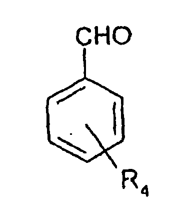 ou 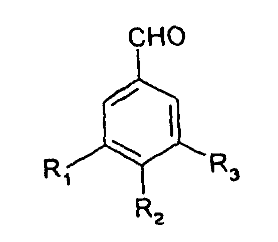
pour former un produit de condensation et la décarboxylation du produit de condensation en présence d'un catalyseur de cuivre pour produire le cis-stilbène;
où les groupes R₁, R₂ et R₃ sont indépendamment sélectionnés parmi alkyle, alcoxy, halogène ou SR, où R est un groupe alkyle substitué ou non substitué; et
R₄ est un substituant ou des substituants à la position 4, ou à la position 3 et à la position 4, ou à la position 3, la position 4 et la position 5; et
le substituant R₄ ou les substituants sont sélectionnés dans les groupes hydrogène, hydroxyle, halogène ou alcoxy.

2. Méthode de la revendication 1, où R₁, R₂ et R₃ sont indépendamment sélectionnés parmi des groupes méthyle, éthyle, méthoxy, éthoxy ou fluoro.

3. Méthode de la revendication 1 ou de la revendication 2 pour la synthèse de la cis-combrestatine-A4, R₁, R₂, et R₃ sont tous méthoxy, et R ₄ est un groupe hydroxyle à la position 3 et un groupe méthoxy à la position 4.

4. Méthode de toute revendication précédente, où la réaction pour produire le produit de condensation est effectuée en présence d'un anhydride d'acide carboxylique et d'une amine tertiaire.

5. Méthode de la revendication 4, où l'anhydride d'acide carboxylique et l'amine tertiaire sont de l'anhydride acétique et de la triéthylamine ajoutés simultanément ou séquentiellement au mélange réactionnel.

6. Méthode de toute revendication précédente, où la réaction pour produire le produit de condensation est entreprise en chauffant les réactifs sous reflux dans un solvant.

7. Méthode de la revendication 6, où la durée de la réaction est comprise entre 1 et 6 heures.

8. Méthode de l'une quelconque des revendications précédentes, où le produit de condensation est recristallisé avant d'effectuer la réaction de décarboxylation.

9. Méthode de l'une quelconque des revendications précédentes, où la réaction de décarboxylation est effectuée par chauffage à une température comprise entre environ 200 et 250°C.

10. Méthode de l'une quelconque des revendications précédentes, où dans la réaction de décarboxylation, le produit de condensation est chauffé en présence du catalyseur de cuivre.

11. Méthode de la revendication 10, où le catalyseur de cuivre est du cuivre en poudre ou un composé de cuivre tel que du triflate de cuivre ou du chromite de cuivre.

12. Méthode de l'une quelconque des revendications 9 à 11, où la réaction de décarboxylation est effectuée dans un solvant à fort point d'ébullition.

13. Méthode de la revendication 12, où le solvant à fort point d'ébullition est une quinoléine, une quinoléine substituée ou une isoquinoléine.

14. Méthode de l'une quelconque des revendications précédentes, où la réaction de décarboxylation utilise un catalyseur de cuivre en poudre dans un solvant tel que la quinoléine à une température d'environ 230°C.

15. Méthode de l'une quelconque des revendications précédentes, qui comprend l'étape initiale de synthétiser l'acide arylacétique ou le benzaldéhyde substitué.

16. Méthode de l'une quelconque des revendications précédentes, comprenant de plus l'étape de faire réagir le stilbène pour former un dérivé, sel ou promédicament.

17. Méthode de l'une quelconque des revendications précédentes comprenant de plus la purification du stilbène ou son dérivé ou son sel.

18. Méthode de la revendication 17, comprenant de plus la formulation du stilbène dans une composition pharmaceutique.
